# EUROPÄISCHE PATENTSCHRIFT

(11) **EP 1 631 277 B1**
(45) Veröffentlichungstag und Bekanntmachung des Hinweises auf die Patenterteilung: **21.01.2009**
(21) Anmeldenummer: 04739625.4
(22) Anmeldetag: 04.06.2004
(51) Int. Cl.: A61K 31/164, C11C 3/00

(54) **DERIVATE NATÜRLICHER, HALBSYNTHETISCHER UND SYNTHETISCHER LIPIDE AUS DER GRUPPE DER CERAMIDE UND DER SPHINGOSINE, ARZNEIMITTEL UND IHRE VERWENDUNG ALS THERAPEUTICA IN DER MEDIZIN, SPEZIELL IN DER DERMATOLOGIE**
NATURAL SEMI-SYNTHETIC AND SYNTHETIC LIPID DERIVATIVES OF CERAMIDE AND SPHINGOSINE GROUPS, DRUGS AND THE MEDICAL USE THEREOF IN THE FORM OF THERAPEUTIC AGENTS IN PARTICULAR FOR DERMATOLOGY
DERIVES DE LIPIDES NATURELS, SEMI-SYNTHETIQUES ET SYNTHETIQUES DU GROUPE DES CERAMIDES ET DES SPHINGOSINES, MEDICAMENTS ET LEUR UTILISATION EN TANT QU'AGENTS THERAPEUTIQUES EN MEDECINE, NOTAMMENT EN DERMATOLOGIE

(30) Priorität: 06.06.2003 DE 10325829
(43) Veröffentlichungstag der Anmeldung: 08.03.2006
(73) Patentinhaber: PLT Patent & Licence Trading Ltd., c/o The B-Net Sheraton House Castle Park Cambridge CB3 0AX (GB)
(72) Erfinder: WOLF, Hans, Uwe, 89231 Neu-Ulm (DE); DORMANN, Jörg, Martin, 89134 Blaustein (DE)
(74) Vertreter: Pfenning, Meinig & Partner GbR
(86) Internationale Anmeldenummer: PCT/EP2004/006076
(87) Internationale Veröffentlichungsnummer: WO 2004/108124

(56) Entgegenhaltungen:
- EP-A- 1 201 736
- WO-A-00/68238
- DE-A- 19 841 794

## Beschreibung

Gegenstand der vorliegenden Erfindung sind neue Substanzen, die sich aus natürlicherweise vorkommenden Ceramiden und Sphingosin sowie aus synthetischen Verbindungen mit prinzipiell gleicher Struktur dadurch ableiten, dass sie Dimere, Trimere, Tetramere, etc., somit also Oligomere der Ausgangssubstanzen darstellen.

Grundsätzlich enthalten alle biologischen Membranen, insbesondere Zellmembranen, als essentielle Bestandteile so genannte Lipide, die strukturell unterschiedlich aufgebaut sind, die sich jedoch in ihrer prinzipiellen Bauweise ähneln. Die prinzipielle Ähnlichkeit der Struktur besteht darin, dass sie aus einem hydrophoben und einem hydrophilen Anteil aufgebaut sind.

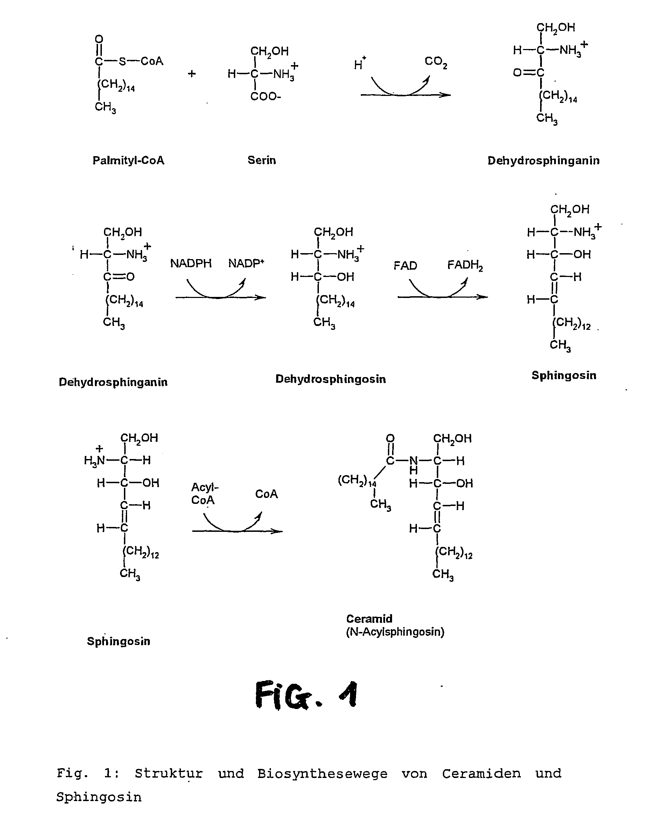

Im Fall der nicht phosphathaltigen Lipide aus der Gruppe der Ceramide und der Sphingosine sowie Lipidanaloger Substanzen besteht der hydrophobe Molekülbereich aus zwei modifizierten Fettsäureresten, die sich beispielsweise von der Palmitinsäure ableiten, während der hydrophile Anteil ein decarboxylierter Serinrest ist. Einer der beiden genannten Fettsäurereste ist über eine Carbonsäureamid-Bindung mit dem Stickstoffatom des modifizierten Serinrests verbunden, während der zweite Fettsäurerest am N-tragenden Kohlenstoffatom des modifizierten Serinrests gebunden ist. Bei dieser Art der Verknüpfung bildet sich formal aus der ehemaligen COOH-Gruppe eine α-ständige OH-Gruppe und es entsteht eine β-γ-ständige Doppelbindung. Fig. 1 zeigt die Struktur und die Biosynthese der Lipide aus der Gruppe der Ceramide und der Sphingosine.

Für die eine genannte Fettsäurekomponente der Sphingosine und für die zwei genannten Fettsäurekomponenten der Ceramide kommen neben der Palmitinsäure (n-Hexadecansäure), C₁₅H₃₁-COOH, als Bausteine der genannten Lipide bzw. Lipid-analogen Substanzen eine große Zahl verschiedener Monocarbonsäuren in Frage, deren Zahl der Ketten-C-Atome zwischen 10 und 40 liegt. Hierzu gehören beispielsweise die gesättigten Monocarbonsäuren n-Dodecansäure (Laurinsäure, C₁₁H₂₃-COOH), n-Tetradecansäure (Myristicinsäure, C₁₃H₂₇-COOH), n-Octadecansäure (Stearinsäure, C₁₇H₃₅-COOH), n-Eicosansäure (Arachinsäure, C₁₉H₃₉-COOH), n-Tetracosansäure (Lignocerinsäure, C₂₃H₄₇-COOH), die einfach ungesättigte *cis*-Δ⁹-Hexadecensäure (Palmitoleinsäure, C₁₅H₂₉-COOH) und *cis*-Δ⁹-Octadecensäure (Oleinsäure, Ölsäure, C₁₇H₃₃-COOH), die doppelt ungesättigte *cis, cis*-Δ⁹-Δ¹²-Octadecadiensäure (Linolsäure, C₁₇H₃₁-COOH), die dreifach ungesättigte all-cis-Δ⁹, Δ¹², Δ¹⁵-Octadecatriensäure (Linolensäure, C₁₇H₂₉-COOH) sowie die einfach hydroxylierte α-Hydroxytetracosansäure (Cerebronsäure, C₂₂H₄₅-CHOH-COOH). Darüber hinaus sind zahlreiche weitere, in der Natur selten vorkommende bzw. synthetisch herstellbare gesättigte oder ungesättigte Monocarbonsäuren wie z. B. die Decansäure (C₁₀H₂₁-COOH), die Octacosansäure (C₂₈H₅₇-COOH) oder die cis-Δ⁹-Octacosansäure (C₂₈H₅₅-COOH) als Strukturkomponenten einsetzbar.

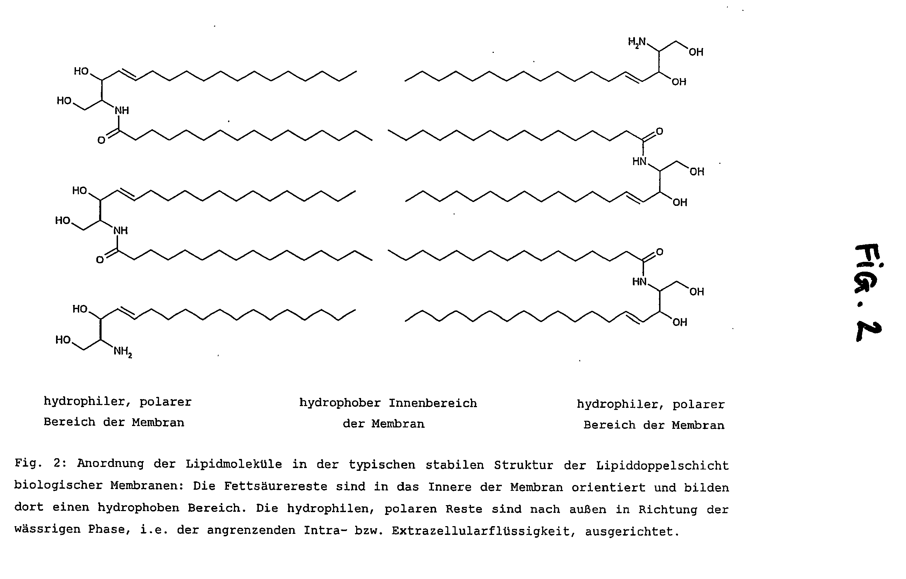

Die amphiphile Struktur der Lipide und Lipid-analogen synthetischen Substanzen, d.h., die gleichzeitige Anwesenheit eines (stark) hydrophoben und eines hydrophilen, polaren Anteils der Molekülstruktur, führt dazu, dass sich die Lipide in einer wässrigen Phase spontan zu einer Lipid-Doppelschicht, einem so genannten "Lipid-Bilayer" anordnen, der u. a. die Grundlage der Struktur biologischer Membranen darstellt. Das Bauprinzip dieser Doppelschicht ist für alle Lipide gleich: Sie ordnen sich in zwei parallelen, eng zusammenliegenden Schichten an, wobei sich jeweils die hydrophoben Reste der Lipid-Moleküle direkt gegenüberliegen und in Kontakt treten. Sie bilden somit den hydrophoben inneren Bereich der Membrandoppelschicht, während die hydrophilen Reste auf beiden Seiten der Lipid-Doppelschicht mit der wässrigen Phase in Kontakt stehen (s. Fig. 2). Die Tendenz zur Ausbildung dieser Lipid-Doppelschicht besteht sowohl innerhalb als auch außerhalb eines Organismus, z. B. in einem wässrigen System, in welchem die Eigenschaften der Lipid-Doppelschichten in eigens hierzu ausgelegten experimentellen Anordnungen untersucht werden können.

Obwohl sich die Struktur der Lipid-Doppelschicht in einem Organismus spontan ausbildet und eine erhebliche Stabilität besitzt, besteht in einem Organismus, z. B. bei Vorliegen einer Lipid-Stoffwechselstörung, die Möglichkeit, dass eine biologische Membran einen Teil ihrer Lipid-Komponenten verliert, weil diese Lipide entweder zu langsam und/oder in einem unzureichenden Ausmaß gebildet oder (zu schnell) verstoffwechselt werden und damit der Membranstruktur entzogen werden, wobei die betreffenden Membranen an den jeweiligen Lipiden verarmen. Dies führt u. a. zu einer Störung der Membranstruktur und -funktion. Ein gut bekanntes Beispiel für derartige Veränderungen ist die Verarmung der Lipid-Doppelschichten des Stratum corneum der menschlichen Haut an Ceramiden und Sphingosinen. Eine derartige Verarmung tritt bei bestimmten Hauterkrankungen nachweislich auf.

Insbesondere auf dem Gebiet der Klinischen Medizin ist es in den genannten Fällen von Erkrankungen wünschenswert, die Struktur der im Organismus vorhandenen biologischen Membran, i. e. der Lipid-Doppelschichten, zu stabilisieren. Dies kann dadurch erfolgen, dass bei therapeutischen Maßnahmen biologische Substanzen verwendet werden, die den folgenden Anforderungen entsprechen:
- Die Struktur der therapeutisch anzuwendenden Substanzen soll derjenigen der Membran-Lipide in ihren Prinzipien entsprechen.
- Die therapeutisch anzuwendenden Substanzen sollen durch die Stoffwechselvorgänge des Organismus, insbesondere durch Enzyme des Lipid-Stoffwechsels, deutlich langsamer abgebaut werden, als dies bei den originären Lipiden der Fall ist.

Die Aufgabe der vorliegenden Erfindung ist es deshalb, neuartige Lipide, die im Aufbau den natürlichen Lipiden möglichst nahe kommen, sowie ein Arzneimittel und eine entsprechende Anwendung in der Medizin anzugeben.

Die Aufgabe wird in Bezug auf die Lipide durch die Merkmale des Patentanspruches 1, in Bezug auf das Arzneimittel durch die Merkmale des Anspruchs 12 und in Bezug auf die Verwendung durch die Merkmale der Patentansprüche 13 und 14 gelöst. Die Unteransprüche zeigen vorteilhafte Weiterbildungen auf.

Die neu vorgeschlagenen Derivate der Lipide bestehen somit aus Oligomeren der Ceramide und/oder Sphingosine. Unter Oligomeren im Sinne der Erfindung wird die Verknüpfung von zwei bis zwölf Monomeren verstanden. Bevorzugt sind hier insbesondere Dimere, Tetramere, Hexamere und Octamere.

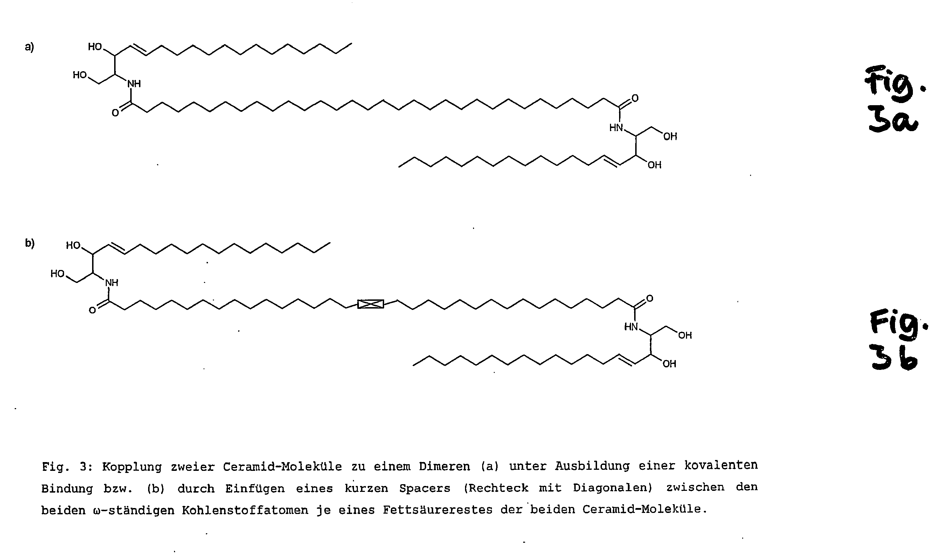

Der Begriff "Dimerisierung" wird in der vorliegenden Beschreibung und den Ansprüchen der Erfindung auch dann verwendet, wenn es sich nicht nur um die direkte Verbindung zweier Moleküle (unter Verdopplung der Zahl der jeweils enthaltenen Atome) handelt, sondern auch dann, wenn die beiden ursprünglichen Einzelmoleküle durch eine kurze Molekülbrücke im Sinne eines sog. "Spacers" verbunden sind (vgl. Fig. 3b).

Die Fettsäurekomponente der Sphingosine und der Ceramide bestehen dabei bevorzugt aus Palmitinsäure oder aus einer weiteren beliebigen Monocarbonsäure mit einer Kettenlänge zwischen 10 und 40 C-Atomen.

Die Fettsäurekomponente ist dabei bevorzugt ausgewählt aus den in Anspruch 3 angegebenen gesättigten Monocarbonsäuren.

Die Verknüpfung der Lipid-Moleküle kann dabei in der "tail-to-tail"-Anordnung jeweils über den hydrophoben Fettsäurerest, vorzugsweise über das ω-ständige Kohlenstoffatom der Fettsäurekette erfolgen, wobei die Verbindung durch eine kovalente Bindung hergestellt wird. Eine zweite Möglichkeit besteht darin, dass statt einer kovalenten Bindung ein so genannter "intradimerer" Spacer mit frei wählbarer Molekülkettenlänge eingesetzt wird. Der intradimere Spacer besteht dabei aus mindestens einem Kohlenstoffatom und/oder mindestens einem Heteroatom wie z. B. Sauerstoff, Stickstoff usw. Bevorzugte Kettenlängen des intradimeren Spacers sind 1-4 Atome.

Auch bei der Verknüpfung der Lipid-Moleküle über die "head-to-head"-Anordnung kann diese über den hydrophilen Strukturanteil durch eine kovalente Bindung erfolgen. Im Falle der "head-to-head"-Verknüpfung ist es erfindungsgemäß als Alternative vorgesehen, einen so genannten "interdimeren Spacer" mit frei wählbarer Molekülkettenlänge und Zusammensetzung einzusetzen. Für den Fall, dass ein intradimerer Spacer eingesetzt wird, ist es bevorzugt, dass dieser überwiegend hydrophil ist. Geeignete Strukturkomponenten für einen derartigen hydrophilen Spacer sind Glycerin, Aminosäuren und/oder Kohlenhydratkomponenten wie Monosaccharide, Disaccharide, Oligosaccharide, etc..

Die Herstellung der erfindungsgemäßen Lipide erfolgt nach den für den Fachmann üblichen bekannten Verfahren.

Die Erfindung wird nachfolgend anhand der Figuren näher erläutert.
- Fig. 1: zeigt die Struktur und den Biosyntheseweg von Ceramiden und Sphingosin.
- Fig. 2: zeigt die Anordnung der Lipid-Moleküle in einer typischen stabilen Struktur der Lipid-Doppelschicht biologischer Membranen.
- Fig. 3a: zeigt die Kopplung zweier Ceramidmoleküle zu einem Dimeren unter Ausbildung einer kovalenten Bindung und

- Fig. 3b: mit einem Spacer.
- Fig. 4: zeigt in schematischer Darstellung die Anordnung eines "head-to-head"-Lipid-Dimeren.

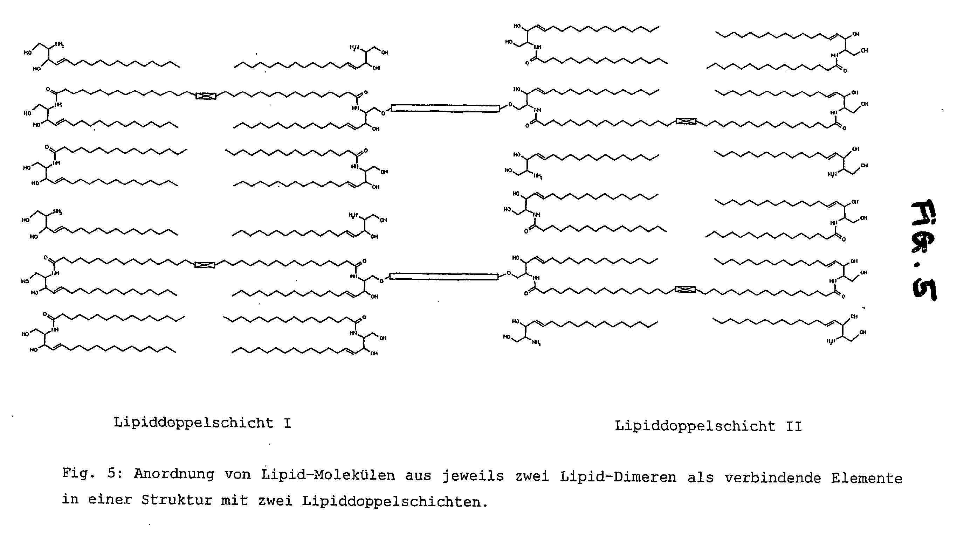

- Fig. 5: zeigt in schematischer Darstellung die Anordnung von Lipid-Molekülen aus jeweils zwei Lipid-Dimeren als verbindende Elemente in einer Struktur mit zwei Lipid-Doppelschichten.

Wegen der strukturellen Asymmetrie der gesamten Gruppe der Lipide - auf der einen Seite der/die Fettsäurerest(e) als hydrophober Strukturanteil (im englischen Sprachgebrauch als "tail" bezeichnet) und auf der anderen Seite der hydrophile Rest (im englischen Sprachgebrauch als "head" bezeichnet) - lassen sich drei prinzipiell verschiedene Arten unterscheiden, wie zwei monomere Lipid-Moleküle zu einem dimeren Lipid-Molekül kovalent verbunden werden können:
1. in Form einer "tail-to-tail"-Anordnung, d. h., durch kovalente Bindung zwischen je einem hydrophoben Fettsäurerest der beiden zu verbindenden Moleküle,
2. in Form einer "head-to-head"-Anordnung, d. h., durch kovalente Bindung zwischen den beiden hydrophilen, polaren Strukturanteilen der beiden zu verbindenden Moleküle,
3. in Form einer "head-to-tail"-Anordnung, d. h., durch kovalente Bindung zwischen dem hydrophilen, polaren Anteil des einen Lipid-Moleküls und einem hydrophoben Fettsäurerest des zweiten Moleküls.

Die Variante 1, i. e. die "tail-to-tail"-Anordnung, basiert auf der Verknüpfung vorzugsweise der ωständigen Kohlenstoffatome der Fettsäurereste der beiden zusammenzufügenden Moleküle. Auf diese Weise entsteht ein Molekül, das sich auf Grund der Anordnung seines hydrophoben Molekülbereichs problemlos in eine biologische Lipid-Doppelschicht integrieren kann. Fig. 3a zeigt die Art der Verknüpfung und die Ähnlichkeit des Dimerisierungsprodukts mit der physiologischen Struktur der Lipid-Doppelmembran am Beispiel zweier Ceramid-Moleküle (vgl. Fig. 2).

Die "tail-to-tail"-Anordnung stellt die unmittelbare Nachahmung der natürlicherweise in den biologischen Membranen vorhandenen stabilen Anordnung der Lipid-Moleküle dar, wie durch einen Vergleich mit der Anordnung der Lipide in Fig. 2 zu erkennen ist. Das "tail-to-tail"-Dimere ist als wesentliche Grundstruktur für die Gesamtheit aller weiteren hier beschriebenen Lipid-Oligomere anzusehen.

Die' Dimerisierung des Ceramids führt somit zu einem Molekül, das sich nicht nur in die Struktur einer Lipid-Doppelmembran ohne Probleme einfügt, sondern das auch darüber hinaus durch die Anwesenheit einer kovalenten Bindung zwischen den ω-Positionen der Fettsäurereste zweier sich gegenüberliegender Lipid-Moleküle zu einer erheblichen Strukturstabilisierung der Lipid-Doppelmembran beiträgt.

Die Variante 2, i. e. das "head-to-head"-Dimere, besitzt eine Struktur, die eine Integration des Moleküls in eine Lipid-Doppelschicht nicht erlaubt, weil der hydrophile Bereich dieses dimeren Moleküls in das hydrophobe Innere der Membrandoppelschicht zu liegen käme, was eine äußerst instabile Struktur darstellen würde, die sich demzufolge nicht spontan ausbildet. Die "head-to-head"-Dimeren haben jedoch insofern eine biologische oder medizinische Bedeutung, als die beiden derart verknüpften Lipid-Moleküle in zwei parallel in nahem Abstand angeordneten Lipid-Doppelschichten verankert sein können, wobei sich jedes der beiden Monomere in jeweils einer Hälfte der beiden parallelen Membranen befindet.

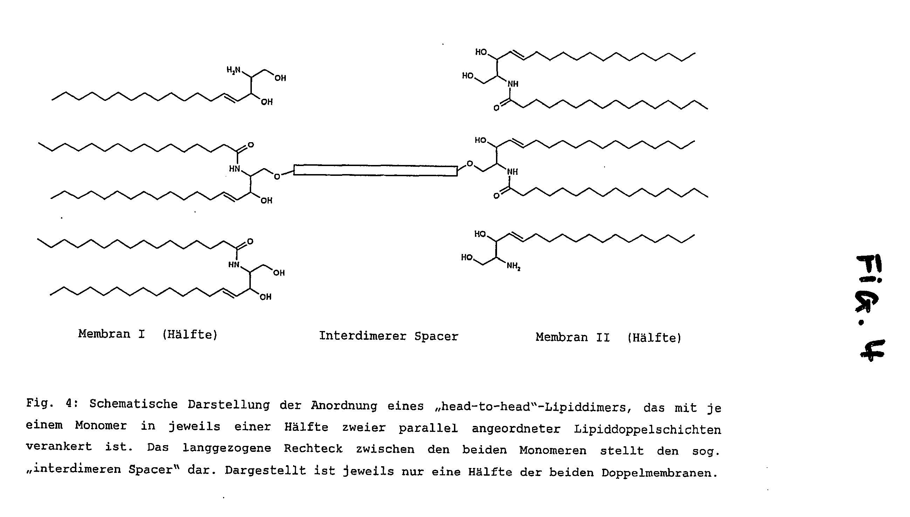

Im Fall des "head-to-head"-Lipid-Dimers kann es allerdings aus räumlichen Gründen erforderlich sein, zwischen die beiden Monomere einen sog. Spacer (mit variabler Kettenlänge) einzubauen, um eine Integration der beiden Lipid-Komponenten in die beiden Lipid-Membranen auch dann zu ermöglichen, wenn diese Membranen einen gewissen Abstand voneinander haben (s. hierzu Fig. 4). Dies trifft z.B. bei den parallel angeordneten Lipid-Doppelschichten des Stratum corneum der menschlichen Haut zu.

Die Variante 3 der Lipid-Dimerisierung hat praktisch keine biologische oder medizinische Bedeutung, da sich ein Molekül dieser Struktur nicht in irgendeiner Weise in eine oder zwei parallel angeordnete biologische Membranen integrieren lässt. In allen Fällen müssten zumindest zum Teil hydrophile Strukturanteile in hydrophobe Bereiche der Membranen integriert werden, was bekanntermaßen zu sehr instabilen Strukturen führen würde, die sich aus diesem Grunde nicht spontan bilden können.

Die Erfindung umfasst weiterhin die Möglichkeit einer Dimerisierung in analoger Weise auch für die biologischen Vorläuferstufen der Ceramide, i. e. für die Sphingosine. Weiterhin ist die Möglichkeit gegeben, Hybridmoleküle aus je einem Molekül Ceramid und einem Molekül Sphingosin herzustellen und in der unten beschriebenen Weise zu therapeutischen Zwecken einzusetzen.

Lipid-Oligomere der beschriebenen Art können in der Medizin zu therapeutischen Zwecken überall dort angewandt werden, wo der natürliche Aufbau biologischer Membranen durch pathologische Vorgänge gestört ist und sich durch den Einsatz dieser oligomeren Verbindungen eine Stabilisierung der Membranstruktur und/oder eine Veränderung der Membraneigenschaften im Sinne eines therapeutischen Ziels (z. B. zur Erhöhung der Membranfluidität) erreicht werden soll.

### Nachfolgend einige Beispiele:

1.) Im Fall bestimmter Vergiftungen, welche vorzugsweise die Leber angreifen, wie z.B. eine Vergiftung mit Tetrachlormethan (Tetrachlorkohlenstoff, "TETRA", CCl₄) werden die Lipide der Leberzellmembranen durch Radikale in ihrer Struktur angegriffen. Bei diesem Vorgang werden die Fettsäurereste der Lipide oxidiert, wodurch nach einer Reihe verschiedener Reaktionen die Kohlenstoffkette abgebaut wird. Die Folge hiervon ist ein partieller Abbau der Lipide und eine Destabilisierung der Membran, die zur einer teilweisen Auflösung der Zellmembran und damit einer schweren Schädigung der Zelle führt. Die Zufuhr der beschriebenen Lipid-Dimere kann in einem derartigen Vergiftungsfall zu einer deutlichen Stabilisierung der Membran der geschädigten Leberzellen beitragen.
2.) Ein weiteres Beispiel ist die Störung der Lipid-Zusammensetzung der Leberzellmembran. Es ist bekannt, dass das in der Leberzelle vorhandene Enzym Ceramid-Synthase durch exogene Gifte wie Mykotoxine aus der Gruppe der Fumonisine gehemmt wird. Dies führt zu einer Verminderung der Ceramid-Synthese und damit zu einer Verminderung des Einbaus von Ceramiden in die Leberzellmembran. Dieser Mangel an Ceramiden.kann durch die beschriebenen Oligomere aufgehoben werden.
3.) Eine Veränderung der Lipid-Zusammensetzung von Nervenzellen tritt bei einer großen Zahl unterschiedlicher pathologischer Schädigungen von Nervenzellen auf. Hierzu gehören u. a. die Neuronopathie, die Axonopathie und die Myelinopathie. Als Ursachen für die Schädigung bzw. den Abbau der Lipid-reichen Myelinscheiden werden exogene Schadstoffe in Betracht gezogen. Die Ursache der Multiplen Sklerose ist bislang noch nicht bekannt. Im Fall von Myelinopathien kommen für eine Stabilisierung der Lipid-Membranen der Myelinscheiden wegen deren spezifischer Struktur vorzugsweise Oligomere der genannten Lipide mit relativ kurzen hydrophoben Spacern zwischen den Monomeren in Betracht.
4.) In mehreren Untersuchungen konnte bisher nachgewiesen werden, dass die Anwesenheit von ω-3-mehrfach-ungesättigten Fettsäuren in Lipiden eine antithrombotische Wirkung besitzt. Hintergrund dieser Wirkung ist offensichtlich die bevorzugte Einlagerung dieser Lipid-Spezies in die Membranen von Blutzellen, insbesondere in Membranen der Blutplättchen, gegenüber derjenigen der Lipide mit ω-6-mehrfach-ungesättigten Fettsäuren. Die Anwendung von Lipid-Dimeren mit einem hohen Gehalt von ω-3-mehrfach-ungesättigten Fettsäuren bietet sich insb. in denjenigen Fällen an, in denen eine genetisch determinierte Fettstoffwechselstörung zu einem hohen thrombotischen, atherosklerotischen und cardiovaskulären Risiko führt.
5.) Nach derzeitiger Kenntnis sind Hauterkrankungen eines der Hauptgebiete für den Einsatz der genannten Lipid-Oligomere aus der Gruppe der Ceramide und der Spingosine, nicht zuletzt deswegen, weil im Stratum corneum der menschlichen Haut diese beiden Lipid-Spezies eine wesentliche Rolle spielen. Daher werden diese Einsatzmöglichkeiten im Abschnitt 3 der vorliegenden Patentschrift ausführlich beschrieben.

Nachfolgend sind einige Beispiele angeführt, welche die Anwendung der erfindungsgemäßen Lipide in der Dermatologie beschreiben.

Verschiedene Hautveränderungen und Hauterkrankungen basieren auf den Veränderungen der Lipid-Zusammensetzung der Stratum-corneum-Schicht in der menschlichen Haut. Diese Veränderungen im Sinne eines Lipid-Verlusts führen zu einer mehr oder weniger stark verminderten Wasserbindefähigkeit der betroffenen Hautpartien.

Hautveränderungen und Hauterkrankungen dieser Art sind beispielsweise (s. hierzu auch Braun-Falco et al., 2002):
1. Atopische Dermatitis
2. "trockene" Haut-Xerose, Xerodermie
3. chronisches kumulativ-toxisches Kontaktekzem
4. alternde Haut
5. durch UV-Licht stark beanspruchte Haut
6. Sebostase
7. Verhornungsstörungen

Wie oben bereits ausgeführt, sind biologische Membranen sehr vieler Zellen aus einer Lipid-Doppelschicht aufgebaut, die eine wirksame Barriere gegenüber dem Extrazellularraum darstellt. Dies trifft auch für das Stratum corneum der Haut zu. Beim Menschen besteht diese Hautstruktur aus mehreren Schichten keratinisierter Corneocyten, die in eine Lipidmatrix einer stark geordneten lamellaren Struktur eingebettet sind. Diese Lipid-Doppelschichten enthalten im Wesentlichen Ceramide, Fettsäuren wie z.B. Palmitinsäure sowie Cholesterol ("Cholesterin").

Gemäß neueren Erkenntnissen zum Pathomechanismus der Atopischen Dermatitis (Arikawa, Ishibashi et al., 2002) und verwandter Erkrankungen (Yarosh, Both und Brown, 2000) ist die Ursache für die Anfälligkeit der Haut im Fall einer derartigen Erkrankung u. a. ein veränderter Lipidstoffwechsel bzw. reduzierter Lipid-Gehalt des Stratum corneum. Diese Veränderungen betreffen u.a. den Ceramid- und Sphingosin-Stoffwechsel. Beispielsweise zeigten sich im Fall der Atopischen Dermatitis in der Haut der Patienten erniedrigte Gehalte u.a. an Ceramid 3, Ceramid 4, ω-Hydroxyceramiden (Macheleidt, Kaiser und Sandhoff, 2002) und Sphingosin. Die letzte der genannten Substanzen besitzt eine deutliche antimikrobielle Wirkung. Ein erniedrigter Gehalt an einer derartigen Substanz kann demzufolge die Anfälligkeit der atopischen Haut gegenüber einer bakteriellen Infektion zwanglos erklären.

Die physiologische Zusammensetzung der Membran-Lipide des Stratum corneum der menschlichen Haut ist allerdings noch aus einem zweiten Grund für die normale Struktur und Funktion der Haut von essentieller Bedeutung. Die Anwesenheit eines ausreichenden Gehalts an diesen Lipiden gewährleistet die uneingeschränkte Fähigkeit der Haut zur Bindung einer physiologischen Menge an Wasser. Der Verlust eines Teils der Stratum-corneum-Lipide führt daher zu einer Einschränkung der Wasserbindefähigkeit, dem so genannten transepidermalen Wasserverlust der Haut. Dies zeigt sich im Auftreten einer "trockenen" und faltigen Haut, die insbesondere, aber nicht ausschließlich, im höheren Lebensalter häufig auftritt.

Die heutigen Möglichkeiten, die Symptome und Folgen der genannten Hautkrankheiten, insb. der Atopischen Dermatitis, zu lindern (von einer Heilung kann derzeit noch keine Rede sein), sind nach wie vor noch sehr begrenzt. Die topische Anwendung spezieller Glucocorticoide und immunsuppressiver Wirkstoffe ist wegen der Toxizität dieser Substanzen mit erheblichen Risiken verbunden. Bestimmte Corticoide verursachen sogar einen geradezu kontraproduktiven Effekt, indem sie zu einem Verlust an Ceramiden, Cholesterol und freien Fettsäuren führen.

Unter Berücksichtigung des heutigen Kenntnisstands über die Bedeutung einer physiologischen Lipid-Zusammensetzung der Stratum-corneum-Membranen ist es folgerichtig, dass versucht wird, die im Stratum corneum vorhandenen Defizite an Membran-Lipiden durch exogene Zufuhr auszugleichen. In der Praxis versucht man daher, mit Hilfe von Salben, Cremes und dergleichen die fehlenden Lipide, im vorliegenden Fall insbesondere die Ceramide, der veränderten bzw. erkrankten Haut zuzuführen. Dies erfolgt beispielsweise durch speziell hierfür formulierte Lipid-Präparate unter Einschluss von Ceramiden, u. a. durch die Verwendung von Liposomen. Zahlreiche Produkte auf der Basis bestehender Patente sind zur Therapie der genannten Hautkrankheiten inzwischen auf dem Markt.

Die hier geschilderten therapeutischen Maßnahmen sind sicherlich als prinzipiell richtig anzusehen, da sie in logischer Weise die vorhandenen Defizite des Stratum corneum an Lipiden auszugleichen versuchen. Die während der letzten Jahre mit diesen therapeutischen Maßnahmen gemachten Erfahrungen zeigen jedoch, dass trotz der prinzipiellen Richtigkeit des therapeutischen Ansatzes die Ergebnisse dieser Heilbehandlungen keineswegs überzeugend sind. Zum Teil ist der Erfolg der durchgeführten Maßnahmen unsicher. Selbst dann, wenn sich ein annähernd akzeptabler Erfolg der Heilbehandlung einstellt, hat eine derartige Heilbehandlung zumindest zwei gravierende Nachteile:
- Das Ausmaß des Heilerfolgs ist nicht so groß, dass von einer vollständigen Gesundung der erkrankten Haut gesprochen werden kann.
- Um einen einigermaßen akzeptablen Heilerfolg an der Haut über einen längeren Zeitraum zu gewährleisten, müssen die genannten Lipide permanent der Haut zugeführt werden.

Beide Nachteile sind auf eine gemeinsame Ursache zurückzuführen. Die genannten Lipide wie die Ceramide und Sphingosine sind keine statischen Komponenten der Haut, sondern sie sind Zwischenprodukte einer Reaktionsfolge, in der z.B. Sphingosin in Ceramid umgewandelt wird, das seinerseits wiederum zu Sphingomyelin, und zu Gangliosiden umgesetzt wird. Die gesamte Reaktionsfolge ist in Fig. 1 dargestellt.

Diese Reaktionsabfolge stellt ein Fließgleichgewicht dar, in welchem eine gewisse Menge der genannten Komponenten durch die Wirkung bestimmter Enzyme von Stufe zu Stufe weiterverarbeitet wird. Es liegt somit ein gewisser Durchsatz an Substanz vor. Die exogen zugeführten Lipide, z. B. Ceramide, werden in diese Reaktionsabfolge eingeschleust. Liegt *a priori* eine Störung dieser Reaktionsabfolge vor, die dann zu einer pathologischen Lipid-Zusammensetzung des Stratum corneum führt, so ist zu erwarten, dass die exogene Zufuhr von Lipiden an diesem pathologischen Zustand grundlegend nichts oder nicht viel ändern kann, da der exogen zugeführte Lipid-Anteil vom Organismus in gleicher Weise weiterverarbeitet wird, wie dies bei dem endogenen Anteil der Lipide der Fall ist. Ein Heilerfolg ist daher mit den derzeit zur Verfügung stehenden therapeutischen Möglichkeiten in hohem Maße davon abhängig, dass die betreffenden Lipide schneller in die Haut eindringen können als sie in die vorhandenen physiologischen Reaktionsschritte eingeschleust werden, und dass sie über einen längeren Zeitraum, im Extremfall lebenslang, kontinuierlich zugeführt werden.

Das vorliegende Problem ist nicht ohne weiteres lösbar. Der Geschwindigkeit der Aufnahme von Lipiden in das Stratum corneum hinein sind gewisse physiologische und physikalisch-chemische bzw. biochemische Grenzen gesetzt, z. B. hinsichtlich der Diffusionsgeschwindigkeit der Lipide. Diese Geschwindigkeit kann nicht beliebig gesteigert werden. Zum anderen sind die an der in Fig. 1 gezeigten Reaktionskaskade beteiligten Enzyme durch exogene Maßnahmen nicht oder nicht ohne gravierende Probleme zu beeinflussen.

Zur Lösung dieses Problems ist es erforderlich, grundsätzlich andere Prinzipien anzuwenden, um die therapeutische Wirksamkeit exogen zugeführter Ersatzsubstanzen zu erhöhen.

Nach gegenwärtiger Kenntnis besteht lediglich eine weitere Möglichkeit des Eingriffs in die oben gezeigte Reaktionsabfolge, um eine bessere und länger an-dauernde Wirksamkeit der zugeführten Ersatz-Lipide zu gewährleisten, i. e. die Abwandlung der Molekülstruktur der zur Therapie eingesetzten Lipide.

Diese Abwandlung der Molekülstruktur muss den folgenden drei Anforderungen genügen.
1. Die grundsätzliche Struktur der eingesetzten Lipide, welche die Ausbildung der Lipid-Doppelmembran, ermöglicht, soll nicht nur erhalten bleiben, sondern die Fähigkeit zur Ausbildung der Doppelmembran soll verstärkt werden, weil die durch die genannten Erkrankungen geschädigte Haut ohnehin nur eine eingeschränkte Fähigkeit zum Aufbau und zum Erhalt der physiologischen Lipid-Doppelmembran besitzt.
2. Die Struktur der eingesetzten Lipide soll bei erhaltener Grundstruktur so verändert werden, dass sie nur noch in geringerem Umfang als Substrate der in der Haut, speziell im Stratum corneum, vorhandenen Enzyme fungieren können. Dies bedeutet, dass sie in deutlich geringerem Umfang als die originären Lipide in die in Fig. 1 gezeigte Reaktionskaskade eingeschleust werden sollen und somit als wesentliche strukturelle Bestandteile des Stratum corneum über einen längeren Zeitraum als die originären Lipide erhalten bleiben sollen.
3. Die Abänderung der Molekülstruktur soll jedoch nur in einem so geringen Umfang erfolgen, dass durch den geringen stoffwechselbedingten Um-oder Abbau der zugeführten oligomeren Zusatz-Lipide solche Substanzen entstehen, die den körpereigenen Lipiden möglichst ähnlich sind. Auf diese Weise wird die Gefahr erheblich verringert, dass Stoffwechselprodukte mit toxischer Wirkung entstehen.

Die genannte Abänderung der Molekülstruktur, welche die drei oben angegebenen Forderungen erfüllt, besteht zunächst in einer Dimerisierung der für die erwartete therapeutische Wirksamkeit eingesetzten Lipide. Diese Dimerisierung muss, um die Anordnung der Lipide in einer Lipid-Doppelmembran nachzuahmen, in der vorstehend beschriebenen Variante 1 erfolgen, i.e. durch Verknüpfung der hydrophoben Fettsäurereste der genannten Lipide.

Durch die Dimerisierung des Moleküls ist die Gewähr gegeben, dass ein derartiges Molekül durch die in der Haut vorhandenen Enzyme des Lipid-Stoffwechsels sehr viel langsamer ab- bzw. umgebaut wird, als dies für die korrespondierenden monomeren Lipide zutrifft. Die mit der Dimerisierung verbundene Vergrößerung des Lipid-Moleküls führt zu einer starken Minderung der enzymatisch gesteuerten Metabolisierung, weil bei der bekanntermaßen hohen Substratspezifität der meisten Enzyme die Größenänderung eines Substrats um den Faktor 2 die Geschwindigkeit des Substratumsatzes erheblich abfallen lässt.

Andererseits sind die entstehenden Reaktionsprodukte von ihrem allgemeinen Aufbau her den natürlicherweise vorkommenden analogen Molekülen des Lipid-Stoffwechsels so ähnlich, dass ein Einschleusen in die entsprechenden Reaktionsabfolgen ohne Probleme möglich ist. Darüber hinaus ist auch in keiner Weise damit zu rechnen, dass die dimerisierten Lipid-Moleküle, wegen der großen Ähnlichkeit mit physiologischerweise vorkommenden Molekülspezies, eine relevante Toxizität besitzen.

Die physiologische Abbaubarkeit des Ceramid-Dimers und anderer dimerer Produkte, die allerdings deutlich geringer sein sollte als die der einfachen Lipid-Moleküle, ist somit eine aus pharmakokinetischen und pharmakologischen Gründen erwünschte Eigenschaft des Moleküls, weil hierdurch die Steuerbarkeit der Therapie besser gewährleistet ist, als wenn keinerlei metabolischer Abbau mehr möglich wäre.

Für den Fall, dass die aus den Ceramiden durch direkte Verknüpfung der beiden ω-ständigen C-Atome der Fettsäurereste entstehenden Produkte eine zu geringe metabolische Abbaubarkeit besitzen, kann die folgende Strukturvariante dadurch zu einer erhöhten Abbaubarkeit führen, dass in das Molekül quasi eine "metabolische Sollbruchstelle" eingeführt wird. Bei dieser Variante wird zwischen die beiden ω-ständigen C-Atome ein so genannter- Spacer eingeführt, der aus einem oder mehreren C-, O- oder N-Atomen besteht (Fig. 3b).

Im einfachsten Fall kann dieser Spacer aus einem Sauerstoffatom bestehen.

Bei einer derartigen Strukturvariante wäre bei der Dimerisierung nicht von dem originären Lipid-Molekül auszugehen, sondern von den ω-Hydroxy-Derivaten, z.B. des Ceramids bzw. analoger Lipid-Moleküle. Die Dimerisierung über das ω-ständige Kohlenstoffatom führt hier nicht zu einer reinen Kohlenwasserstoffkette, wie in Fig. 3a gezeigt, sondern zu einer Sauerstoffverbrückung, wobei aus den beiden ursprünglichen Hydroxylgruppen eine Brücke entsteht, die aus einem Äther-Sauerstoffatom besteht.

...-CH₂-CH₂-OH + HO-CH₂-CH₂-... → ...-CH₂-CH₂-O-CH₂-CH₂--... + H₂O

Das nun entstandene dimere Molekül enthält an der Verbrückungsstelle ein Sauerstoffatom. Die in Nachbarschaft stehenden Kohlenstoffatome (die originären ω-C-Atome) sind nun gegenüber einer Hydroxylierung, etwa durch die Cytochrom-P₄₅₀-abhängigen mischfunktionellen Monooxygenasen, besonders empfindlich. Eine derartige, in unmittelbarer Nachbarschaft zum O-Atom stattfindende Hydroxylierung führt zur Bildung instabiler Verbindungen mit Halbacetalstruktur, die in die entsprechenden Reaktionsprodukte zerfallen. Das eine Reaktionsprodukt mit ω-ständiger OH-Gruppe ist identisch mit dem Ausgangsprodukt ω-Hydroxyceramid. Das andere Reaktionsprodukt ist ein Ceramid mit ω-ständiger Aldehydfunktion, die zur Carbonsäuregruppe weiteroxidiert wird. Damit wird offensichtlich, dass durch einen (verlangsamt ablaufenden) biochemischen Abbau der beschriebenen dimeren Moleküle z. T. die physiologischen Ausgangsverbindungen selbst und z. T. den Ausgangsverbindungen sehr ähnlich gebaute Moleküle entstehen.

Aus Gründen der Übersicht sollen im weiteren Verlauf die als Verbrückung neu hinzugekommenen Molekülteile als "intradimere Spacer" bezeichnet werden. Im hier diskutierten Fall wäre somit das Brückensauerstoffatom ein derartiger intradimerer Spacer.

Für den Fall, dass die Dimerisierung der beiden Lipid-Moleküle gleichzeitig mit einer weiteren kontrollierten Erhöhung der Abbaubarkeit des entstehenden dimeren Moleküls einhergehen soll oder/und dass - etwa aus sterischen Gründen - das entstehende dimere Molekül eine längere Kette haben soll als der Summe der Kettenlängen der monomeren Moleküle entspricht, kann ein längerer intradimerer Spacer zwischen die beiden Fettsäurereste eingebaut werden. Dies wird z.B. durch die Verwendung von Glycolen, im einfachsten Fall von Ethylenglycol, zur Verbrückung von ω-Hydroxyceramiden erreicht. In diesem Fall entsteht ein Reaktionsprodukt, das zwei Sauerstoffatome in der Gesamtkette enthält:

... -CH₂-CH₂-O-CH₂-CH₂-O-CH₂-CH₂- ...

Das Gesamtmolekül ist damit gegenüber der Summe der beiden monomeren Moleküle praktisch um die Länge des intradimeren Spacers -O-CH₂-CH₂-O- gewachsen. Durch die beiden in dieser Kette vorhandenen Sauerstoffatome wird durch die verstärkte Oxidationsfähigkeit die Abbaugeschwindigkeit des Lipid-Dimeren im allgemeinen erhöht.

Auf diese Weise kann durch die Wahl eines geeigneten intradimeren Spacers sowohl die Gesamtgröße des entstehenden dimeren Moleküls als auch das Ausmaß seiner biochemischen Abbaubarkeit frei gewählt werden, weil es möglich ist, sog. "metabolische Sollbruchstellen" in den intradimeren Spacer einzubauen. Allerdings ist darauf zu achten, dass der intradimere Spacer überwiegend lipophile Eigenschaften besitzt, da sonst die Möglichkeit der Integration in die Lipid-Doppelmembran gestört werden könnte.

Ein wesentlicher Aspekt der Pathogenese der oben genannten Hautveränderungen bzw. Hauterkrankungen ist die reduzierte Wasserbindefähigkeit des Hautgewebes, insbesondere im Bereich des Stratum corneum. Physiologischerweise wird das Wasser nicht innerhalb, sondern, da mehrere parallel angeordnete Lipid-Schichten vorliegen, zwischen die einzelnen Lipid-Doppelschichten eingelagert. Dies liegt in der Tatsache begründet, dass das Innere der Lipid-Doppelschicht aus den stark hydrophoben Fettsäureresten aufgebaut ist, während das Medium außerhalb der Lipid-Doppelschicht hydrophiler Natur ist. Eine Speicherung von Wasser in den hydrophoben inneren Bereichen der Lipid-Doppelmembran ist praktisch nicht möglich.

Die oben genannten Hautveränderungen und -erkrankungen sind letztlich auf den Verlust eines Teiles der parallel angeordneten Lipid-Doppelschichten und der zwischen diesen Doppelschichten angeordneten hydrophilen Zwischenschichten sowie von Wasser zurückzuführen. Ziel der therapeutischen Maßnahmen bei diesen Erkrankungen ist somit nicht nur der Wiederaufbau und die Stabilisierung der Lipid-Doppelschichten selbst, wie dies mit Hilfe der oben beschriebenen Dimere der Ceramide oder der Sphingosine erfolgt, sondern weiterhin auch der Aufbau und die Stabilisierung der multilamellaren Lipid-Strukturen mit den dazwischen liegenden hydrophilen Zwischenschichten, die letztendlich für die Wasserbindefähigkeit der Haut von ausschlaggebender Bedeutung sind.

Dieses Ziel wird dadurch erreicht, dass mindestens zwei der oben erwähnten Lipid-Dimere kovalent verknüpft werden. Im Gegensatz zu der oben beschriebenen Bildung dimerer Lipid-Moleküle durch Schaffung einer kovalenten Bindung im hydrophoben Bereich des Moleküls, i. e. am ω-ständigen C-Atom der Fettsäurekette, erfolgt die kovalente Verbindung zweier Lipid-Dimere nach einem anderen Prinzip.
1. Die Verknüpfung zweier Lipid-Dimere erfolgt an den hydrophilen Enden der betreffenden Moleküle Ceramid oder Sphingosin. Wie die Darstellung dieser beiden Substanzen in Fign. 1 und 2 zeigt, steht am hydrophilen Ende des Moleküls jeweils eine OH-Gruppe zur Verfügung, an welcher der Aufbau größerer Moleküle, bestehend aus mindestens zwei Lipid-Dimeren erfolgen kann.
2. Die Verknüpfung zweier Lipid-Dimerer erfolgt nicht direkt, was z.B. bei Ceramiden unter Wasseraustritt durch Ausbildung einer Athersauerstoff-Brücke möglich wäre. Vielmehr ist es aus physiologischen Gründen notwendig, zwischen jeweils zwei sich ausbildenden parallel angeordneten Lipid-Schichten einen Zwischenraum einer definierten Mindestgröße entstehen zu lassen, in welchen sich Wasser und ggf. hydrophile Moleküle, möglicherweise auch das vergleichsweise große Molekül Collagen, einlagern können. Der Aufbau eines Zwischenraumes ist jedoch dann und nur dann möglich, wenn die beiden zu verknüpfenden Lipid-Dimere durch einen Spacer auf Abstand gehalten werden, der im folgenden wegen seiner Anordnung zwischen den dimeren Lipid-Molekülen als "interdimerer Spacer" bezeichnet werden soll (Fig. 4).

Aus den oben erwähnten Gründen- der durch den Spacer geschaffene Zwischenraum zwischen zwei parallel angeordneten Lipid-Lamellen soll Wasser und hydrophile Moleküle aufnehmen und speichern können - sollte der interdimere Spacer stets hydrophile Eigenschaften besitzen. Er kann jedoch je nach vorliegender oben erwähnter Hauterkrankung mit stark unterschiedlicher Kettenlänge ausgestattet sein.

Im folgenden sollen einige Strukturbeispiele von Spacer-Strukturen gegeben werden, bei denen jeweils ein hydrophiler Molekülbaustein mit unterschiedlicher Struktur und Kettenlänge mit der jeweils am hydrophilen Ende des Ceramid- oder des Sphingosin-Moleküls vorhandenen OH-Gruppe verknüpft ist (in diesen Fällen ist jeweils zusätzlich die -CH₂-O-Gruppe der beiden Lipid-Komponenten angegeben):

Mit Glycerin als Spacer-bildendem Molekül ergibt sich die folgende Struktur des intradimeren Spacers mit einer zweifachen Äthersauerstofffunktion:

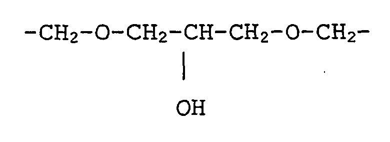

Der Einbau einer zweibasischen Aminosäure wie Asparaginsäure führt unter Ausbildung zweier Estergruppierungen zu einem stark hydrophilen Spacer:

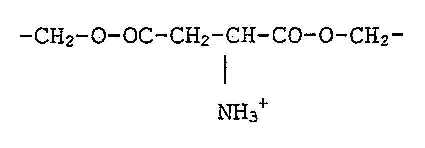

Von besonderem Interesse ist der Aufbau eines Harnstoff-Derivats als Strukturelement des interdimeren Spacers. Dies ist z.B. in Kombination mit zwei Molekülen einer beliebigen hydrophilen Aminosäure, insbesondere einer dibasischen Aminosäure wie Glutaminsäure, möglich. Hierbei entsteht ein besonders langer Spacer mit stark hydrophiler Natur, die u. a. durch die Anwesenheit der beiden negativ geladenen Carboxylgruppen gegeben ist:

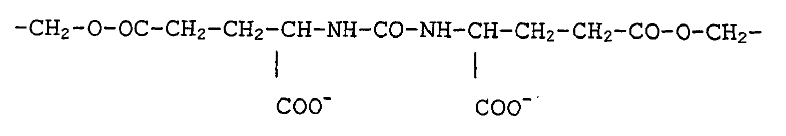

Der Aufbau einer Harnstoff-ähnlichen Struktur ist deswegen von besonderem Interesse, weil Harnstoff über ein sehr hohes Wasserbindevermögen verfügt, was heute bereits in Form Harnstoff-haltiger Salben zur Therapie von Hauterkrankungen genutzt wird, bei denen ein Austrocknen der Haut ein wesentliches Krankheitsmerkmal darstellt.

Der Vielfalt der Spacer-Strukturen und der Länge der verwendbaren Spacer sind praktisch keine Grenzen gesetzt. Die Struktur wie auch die Kettenlänge können je nach Bedarf der speziellen Therapieanforderungen in weiten Bereichen variiert werden. Möglich ist u. a. auch der Einbau bestimmter Monosaccharide wie z.B. von Glucose, was wiederum zu Derivaten physiologischer Substanzen führt. So stellt auch das aus Glucose und Ceramid entstehende Glucosylceramid eine physiologische Substanz dar (Ponce, Witte, et al., 2001; Vielhaber, Pfeiffer, et al., 2001). Weitere aus chemischen und biochemischen Gründen geeignete Komponenten zum Aufbau des interdimeren Spacers sind z. B. Mevalonsäure und Pyrrolidoncarbonsäure, weil es sich hierbei um (annähernd) physiologische Verbindungen handelt, die darüber hinaus z. T. in den Stoffwechsel von Ceramiden im Sinne eines Synthesestimulans eingreifen (Haratake, Ikenaga et al., 2000; Aioi, Okuda et al., 2001).

Die Wahl der genannten verschiedenen Strukturen im interdimeren Spacer führt zu unterschiedlichen biologischen Stabilitäten und damit zu einem unterschiedlichen Ausmaß der gewünschten Abbaubarkeit des oligomeren Lipid-Moleküls, die einerseits unter dem Wert für die monomeren Lipid-Moleküle liegen soll, andererseits jedoch nicht völlig fehlen soll. Damit ist auch über diese intermolekulare Spacerstruktur eine gewisse Steuerbarkeit der Wirkungsstärke und -dauer der zur Therapie eingesetzten Lipid-Oligomere gegeben.

Auch beim metabolischen Abbau der beschriebenen oligomeren Lipid-Moleküle, insbesondere ihrer Spacer, entstehen Abbauprodukte, die identisch mit physiologischen Substanzen sind (z.B. Aminosäuren + monomere Lipide) oder eine sehr große Ähnlichkeit mit ihnen besitzen, so dass die Wahrscheinlichkeit unerwünschter Nebenwirkungen, i.e. toxischer Wirkungen, denkbar gering ist.

Neben der essentiellen Wirkung der Ceramide besitzt diese Substanzgruppe noch eine weitere Wirkung. Sie ist ein Aktivator von Reaktionen, die zum programmierten Zelluntergang (Apoptose) führen (Geilen, Barz und Bektas, 2001). Diese Wirkung macht sich jedoch offensichtlich erst bei hohen intrazellulären Ceramid-Konzentrationen bemerkbar. Da die hier diskutierten Wirkstoffe ohnehin mindestens Ceramid-Dimere darstellen, kann von ihnen keine direkte Wirkung im Sinne einer Apoptose ausgehen, da sie hierfür erst in die Monomeren umgewandelt werden müssten. Dieser Abbau findet jedoch, wie oben bereits beschrieben, entsprechend gesteuert nur in einem geringen Umfang statt.

Gegenüber den dimeren Lipid-Molekülen weisen oligomere Moleküle mit 2-12 Monomeren, insb. Tetramere, Hexamere oder Octamere, eine größere Fähigkeit auf, die Struktur der parallel angeordneten Lipid-Membran-Doppelschichten zu stabilisieren. Durch diese Verbindungen kommt es zum Aufbau von 2 parallelen Lamellen im Fall der Tetrameren, von 3 parallelen Lamellen im Fall der Hexameren, von 4 parallelen Lamellen im Fall der Octameren, etc., mit einer verstärkten Tendenz zur Einlagerung von Wasser und hydrophilen Molekülen unterschiedlichster Größe in die Räume zwischen die parallelen Lamellen.

Oligomere Lipide mit einer ungeraden Zahl von Lipid-Molekülen, im einfachsten Fall also ein trimeres Molekül mit einem intradimeren und einem interdimeren Spacer, sind für die hier angesprochenen Zwecke durchaus auch einsetzbar, wenn sie auch nicht über die optimalen Eigenschaften zur Integration in die vorhandenen Lipid-Bilayer verfügen. Im Beispiel des trimeren Lipid-Moleküls würden sich die beiden über einen intradimeren Spacer verbundenen Lipid-Moleküle in eine Lipid-Doppelschicht optimal integrieren, während das über einen interdimeren Spacer verbundene weitere monomere Lipid-Molekül lediglich in die eine Hälfte der nächsten Lipid-Doppelschicht hineinragt.

Einen Sonderfall stellt ein Lipid-Dimeres dar, das über einen interdimeren Spacer verbunden ist (analog zur oben angegebenen Variante 2 der Verknüpfung bei der Dimerisierung). Auch für diese Moleküle gilt, dass sie für die hier angesprochenen Zwecke durchaus einsetzbar sind, wenn sie auch noch weniger über die optimalen Eigenschaften zur Integration in die vorhandenen Lipid-Bilayer verfügen. In diesem Fall ragen beide vorhandene Lipid-Moleküle lediglich in eine Hälfte der jeweils benachbarten Lipid-Doppelschichten hinein.

## Patentansprüche

1. Derivate natürlicher, halbsynthetischer und synthetischer Lipide,
**dadurch gekennzeichnet ,**
**dass** diese Derivate aus Oligomeren der Lipide ausgewählt aus Ceramiden und/oder Sphingosinen bestehen.

2. Derivate natürlicher, halbsynthetischer und synthetischer Lipide nach Anspruch 1, **dadurch gekennzeichnet, dass** die Fettsäurekomponente der Sphingosine und die Fettsäurekomponenten der Ceramide aus Palmitinsäure (n-Hexadecansäure, C₁₅H₃₁-COOH) oder aus einer anderen Monocarbonsäure mit einer Kettenlänge zwischen 10 und 40 C-Atomen bestehen.

3. Derivate natürlicher, halbsynthetischer und synthetischer Lipide nach Anspruch 1 und 2, **dadurch gekennzeichnet, dass** die Fettsäurekomponenten aus den gesättigten Monocarbonsäuren n-Dodecansäure (Laurinsäure, C₁₁H₂₃-COOH), n-Tetradecansäure (Myristinsäure, C₁₃H₂₇-COOH), n-Octadecansäure (Stearinsäure, C₁₇H₃₅-COOH), n-Eicosansäure (Arachinsäure, C₁₉H₃₉-COOH), n-Tetracosansäure (Lignocerinsäure, C₂₃H₄₇-COOH), *cis*-Δ⁹-Hexade-censäure (Palmitoleinsäure, C₁₅H₂₉-COOH), *cis*-Δ⁹-Octadecensäure (Oleinsäure, Ölsäure, C₁₇H₃₃-COOH), *cis,cis*-Δ⁹-Δ¹²-Octadecadiensäure (Linolsäure, C₁₇H₃₁-COOH), all-*cis*-Δ⁹, Δ¹², Δ¹⁵-Octade-catriensäure (Linolensäure, *C₁₇H₂₉-COOH), α-Hydroxytetracosansäure (Cerebronsäure, C₂₂H₄₅-CHOH-COOH) oder aus Decansäure (C₁₀H₂₁-COOH), Octacosansäure (C₂₈H₅₇-COOH) oder der *cis*-Δ⁹-Octacosansäure (C₂₈H₅₅-COOH) ausgewählt sind.

4. Derivate natürlicher, halbsynthetischer und synthetischer Lipide nach Anspruch 1 bis 3, **dadurch gekennzeichnet, dass** innerhalb des oligomeren Lipid-Moleküls die Verknüpfung von jeweils zwei benachbarten Lipid-Monomeren strikt abwechselnd entweder in der "tail-to-tail"-Anordnung oder in der "head-to-head"-Anordnung erfolgt.

5. Derivate natürlicher, halbsynthetischer und synthetischer Lipide nach den Ansprüchen 1 bis 4, **dadurch gekennzeichnet, dass** zwei benachbarte Lipid-Moleküle in der "tail-to-tail"-Anordnung jeweils über ihren hydrophoben Fettsäurerest, vorzugsweise über das ω-ständige Kohlenstoffatom der Fettsäurekette, durch eine kovalente Bindung verbunden sind.

6. Derivate natürlicher, halbsynthetischer und synthetischer Lipide nach den Ansprüche 1 bis 5, **dadurch gekennzeichnet, dass** zwei benachbarte Lipid-Moleküle in der "tail-to-tail"-Anordnung jeweils über einen so genannten "intradimeren Spacer" mit frei wählbarer Molekülkettenlänge und Zusammensetzung verbunden sind.

7. Derivate natürlicher, halbsynthetischer und syn-thetischer Lipide nach Anspruch 6, **dadurch gekennzeichnet, dass** der intradimere Spacer aus mindestens einem Kohlenstoffatom und/oder mindestens einem Heteroatom (Sauerstoff, Stickstoff, etc.) besteht.

8. Derivate natürlicher, halbsynthetischer und synthetischer Lipide nach den Ansprüchen 1 bis 7, **dadurch gekennzeichnet, dass** zwei benachbarte Lipid-Moleküle in der "head-to-head"-Anordnung jeweils über ihren hydrophilen Strukturanteil miteinander verbunden sind.

9. Derivate natürlicher, halbsynthetischer und synthetischer Lipide nach den Ansprüchen 1 bis 8, **dadurch gekennzeichnet, dass** zwei benachbarte Lipid-Molküle in der "head-to-head"-Anordnung über einen so genannten "interdimeren Spacer" mit frei wählbarer Molekülkettenlänge und Zusammensetzung verbunden sind.

10. Derivate natürlicher, halbsynthetischer und synthetischer Lipide nach Anspruch 9, **dadurch gekennzeichnet, dass** der zwischen den beiden in der "head-to-head"-Anordnung verknüpften Lipid-Dimeren befindliche Spacer überwiegend hydrophil ist.

11. Derivate natürlicher, halbsynthetischer und synthetischer Lipide nach den Ansprüchen 9 oder 10, **dadurch gekennzeichnet, dass** der zwischen den beiden in der "head-to-head"-Anordnung verknüpften Lipid-Dimeren befindliche Spacer als Strukturkomponenten z. B. Glycerin, Aminosäuren und/oder Kohlenhydratkomponenten (Monosaccharide, Disaccharide, Oligosaccharide, etc.) und/oder weitere Strukturkomponenten wie z. B. Mevalonsäure oder Pyrrolidoncarbonsäure enthält.

12. Arzneimittel enthaltend als Wirkstoff Lipide nach mindestens einem der Ansprüche 1 bis 11.

13. Verwendung der Lipide nach mindestens einem der Ansprüche 1 bis 11 zur Herstellung eines Arzneimittels für Erkrankungen, bei denen eine Störung der Lipidzusammensetzung der Zellmembranen eines Organismus hinsichtlich ihres Gehalts an Ceramiden und Sphingosinen vorliegt.

14. Verwendung der Lipide nach mindestens einem der Ansprüche 1 bis 11 zur Herstellung eines Arzneimittels zur Behandlung von Erkrankungen, bei denen eine Störung der Zusammensetzung der Lipid-Doppelschichten des Stratum corneum der Haut hinsichtlich ihres Gehaltes an Ceramiden und Sphingosinen vorliegt.

## Claims

1. Derivatives of natural, semi-synthetic and synthetic lipids,
**characterized in that**
these derivatives comprise oligomers of the lipids selected from ceramides and/or sphingosines.

2. Derivatives of natural, semisynthetic and synthetic lipids according to claim 1, **characterized in that** the fatty acid component of the sphingosines and the fatty acid components of the ceramides comprise palmitic acid (n-hexadecanoic acid, C₁₅H₃₁-COOH) or another monocarboxylic acid having a chain length of between 10 and 40 C atoms.

3. Derivatives of natural, semisynthetic and synthetic lipids according to claim 1 and 2, **characterized in that** the fatty acid components are selected from the saturated monocarboxylic acids n-dodecanoic acid (lauric acid, C₁₁H₂₃-COOH), n-tetradecanoic acid (myristic acid, C₁₃-H₂₇-COOH), n-octadecanoic acid (stearic acid, C₁₇H₃₅-COOH), n-eicosanoic acid (arachic acid, C₁₉H₃₉-COOH), n-tetracosanoic acid (lignoceric acid, C₂₃H₄₇-COOH), *cis*-Δ⁹-hexadecenoic acid (palmitoleic acid, C₁₅H₂₉-COOH), *cis*-Δ⁹-octadecenoic acid (oleic acid, C₁₇H₃₃-COOH), *cis,cis-*Δ⁹-Δ¹²-octadecadienoic acid (linoleic acid, C₁₇H₃₁-COOH), all-*cis*-Δ⁹,Δ¹²,Δ¹⁵-octadecatrienoic acid (linolenic acid, C₁₇H₂₉-COOH), α-hydroxytetracosanoic acid (cerebronic acid, C₂₂H₄₅-CHOH-COOH) or from decanoic acid (C₁₀H₂₁-COOH), octacosanoic acid (C₂₈H₅₇-COOH) or *cis*-Δ⁹-octacosanoic acid (C₂₈H₅₅-COOH).

4. Derivatives of natural, semisynthetic and synthetic lipids according to claim 1 to 3, **characterized in that** within the oligomeric lipid molecule the linkage of in each case two adjacent lipid monomers is strictly alternating either in the "tail-to-tail" arrangement or in the "head-to-head" arrangement.

5. Derivatives of natural, semisynthetic and synthetic lipids according to claims 1 to 4, **characterized in that** two adjacent lipid molecules in the "tail-to-tail" arrangement are in each case bonded by a covalent bond via their hydrophobic fatty acid radical, preferably via the carbon atom in the ω-position of the fatty acid chain.

6. Derivatives of natural, semisynthetic and synthetic lipids according to claims 1 to 5, **characterized in that** two adjacent lipid molecules in the "tail-to-tail" arrangement are in each case bonded via a so-called "intradimeric spacer" with a molecular chain length and composition which can be chosen freely.

7. Derivatives of natural, semisynthetic and synthetic lipids according to claim 6, **characterized in that** the intradimeric spacer comprises at least one carbon atom and/or at least one hetero atom (oxygen, nitrogen etc.).

8. Derivatives of natural, semisynthetic and synthetic lipids according to claims 1 to 7, **characterized in that** two adjacent lipid molecules in the "head to head" arrangement are in each case bonded to one another via their hydrophilic structural content.

9. Derivatives of natural, semisynthetic and synthetic lipids according to claims 1 to 8, **characterized in that** two adjacent lipid molecules in the "head-to-head" arrangement are bonded via a so-called "interdimeric spacer" with a molecular chain length and composition which can be chosen freely.

10. Derivatives of natural, semisynthetic and synthetic lipids according to claim 9, **characterized in that** spacer between the two lipid dimers linked in the "head-to-head" arrangement is predominantly hydrophilic.

11. Derivatives of natural, semisynthetic and synthetic lipids according to claims 9 or 10, **characterized in that** the spacer between the two lipid dimers linked in the "head-to-head" arrangement contains as structural components e.g. glycerol, amino acids and/or carbohydrate components (monosaccharides, disaccharides, oligosaccharides etc.) and/or further structural components, such as e.g. mevalonic acid or pyrrolidonecarboxylic acid.

12. Medicaments containing as the active compound lipids according to at least one of claims 1 to 11.

13. Use of the lipids according to at least one of claims 1 to 11 for the preparation of a medicament for diseases with which a disorder in the lipid composition of the cell membranes of an organism with respect to its content of ceramides and sphingosines exists.

14. Use of the lipids according to at least one of claims 1 to 11 for the preparation of a medicament for treatment of diseases with which a disorder in the lipid double layers of the stratum corneum of the skin with respect to their content of ceramides and sphingosines exists.

## Revendications

1. Dérivés de lipides naturels, semi-synthétiques et synthétiques, **caractérisés en ce que** ces dérivés consistent en oligomères des lipides choisis parmi les céramides et/ou les sphingosines.

2. Dérivés de lipides naturels, semi-synthétiques et synthétiques selon la revendication 1, **caractérisés en ce que** le composant acide gras des sphingosines et les composants acide gras des céramides consistent en acide palmitique (acide n-hexadécanoïque, C₁₅H₃₁-COOH) ou en un autre acide monocarboxylique ayant une longueur de chaîne comprise entre 10 et 40 atomes de carbone.

3. Dérivés de lipides naturels, semi-synthétiques et synthétiques selon les revendications 1 et 2, **caractérisés en ce que** les composants acide gras sont choisis parmi les acides monocarboxyliques saturés acide n-dodécanoïque (acide laurique, C₁₁H₂₃-COOH), acide n-tétradécanoïque (acide myristique C₁₃H₂₇-COOH), acide n-octadécanoïque (acide stéarique, C₁₇H₃₅-COOH), acide n-eicosanoïque (acide arachidique, C₁₉H₃₉-COOH), acide n-tétracosanoïque (acide lignocérique, C₂₃H₄₇-COOH), acide *cis*-Δ⁹-hexadécénoïque (acide palmitoléique, C₁₅H₂₉-COOH), acide *cis-*Δ⁹-octadécénoïque (acide oléique, C₁₇H₃₃-COOH), acide *cis,cis-*Δ⁹-Δ¹²-octadécadiénoïque (acide linoléique, C₁₇H₃₁-COOH), acide tout-*cis*-Δ⁹,Δ¹²,Δ¹⁵-octadécatriénoïque (acide linolénique, C₁₇H₂₉-COOH), acide α-hydroxytétracosanoïque (acide cérébronique, C₂₂H₄₅-CHOH-COOH) ou parmi l'acide décanoïque (C₁₀H₂₁-COOH), l'acide octacosanoïque (C₂₈H₅₇-COOH) ou l'acide *cis*-Δ⁹-octacosanoïque (C₂₈H₅₅-COOH).

4. Dérivés de lipides naturels, semi-synthétiques et synthétiques selon les revendications 1 à 3, **caractérisés en ce qu'**à l'intérieur de la molécule lipidique oligomère la liaison entre chaque fois deux monomères lipidiques contigus s'effectue strictement alternativement soit dans la disposition "tail-to-tail" (queue à queue), soit dans la disposition "head-to-head" (tête à tête).

5. Dérivés de lipides naturels, semi-synthétiques et synthétiques selon les revendications 1 à 4, **caractérisés en ce que** deux molécules lipidiques contiguës dans la disposition "tail-to-tail" sont liées par une liaison covalente, chacune par leur reste d'acide gras hydrophobe, de préférence par l'atome de carbone en position ω de la chaîne de l'acide gras.

6. Dérivés de lipides naturels, semi-synthétiques et synthétiques selon les revendications 1 à 5, **caractérisés en ce que** deux molécules lipidiques contiguës dans la disposition "tail-to-tail" sont liées chacune par un dénommé "espaceur intradimère" de composition et longueur de chaîne de la molécule pouvant être librement choisies.

7. Dérivés de lipides naturels, semi-synthétiques et synthétiques selon la revendication 6, **caractérisés en ce que** l'espaceur intradimère est constitué d'au moins un atome de carbone et/ou d'au moins un hétéroatome (oxygène, azote, etc.).

8. Dérivés de lipides naturels, semi-synthétiques et synthétiques selon les revendications 1 à 7, **caractérisés en ce que** deux molécules lipidiques contiguës dans la disposition "head-to-head" sont liées l'une à l'autre chacune par leur partie structurale hydrophile.

9. Dérivés de lipides naturels, semi-synthétiques et synthétiques selon les revendications 1 à 8, **caractérisés en ce que** deux molécules lipidiques contiguës dans la disposition "head-to-head" sont liées par un dénommé "espaceur intradimère" de composition et longueur de chaîne de la molécule pouvant être librement choisies.

10. Dérivés de lipides naturels, semi-synthétiques et synthétiques selon la revendication 9, **caractérisés en ce que** l'espaceur se trouvant entre les deux dimères lipidiques liés dans la disposition "head-to-head" est essentiellement hydrophile.

11. Dérivés de lipides naturels, semi-synthétiques et synthétiques selon la revendication 9 ou 10, **caractérisés en ce que** l'espaceur se trouvant entre les deux dimères lipidiques liés dans la disposition "head-to-head" contient en tant que composants structuraux, par exemple, du glycérol, des acide aminés et/ou des composants glucidiques (monosaccharides, disaccharides, oligosaccharides, etc.) et/ou d'autres composants structuraux, comme par exemple de l'acide mévalonique ou de l'acide pyrrolidonecarboxylique.

12. Médicament contenant en tant que substance active des lipides selon au moins l'une quelconque des revendications 1 à 11.

13. Utilisation des lipides selon au moins l'une quelconque des revendications 1 à 11, pour la fabrication d'un médicament pour des maladies dans lesquelles est présent un trouble de la composition lipidique des membranes cellulaires d'un organisme, eu égard à leur teneur en céramides et sphingosines.

14. Utilisation des lipides selon au moins l'une quelconque des revendications 1 à 11, pour la fabrication d'un médicament destiné au traitement de maladies dans lesquelles est présent un trouble de la composition des doubles couches lipidiques de la couche cornée de la peau, eu égard à leur teneur en céramides et sphingosines.
